# EUROPEAN PATENT SPECIFICATION

(11) **EP 3 447 483 B1**
(45) Date of publication and mention of the grant of the patent: **12.01.2022**
(21) Application number: 18190759.3
(22) Date of filing: 24.08.2018
(51) Int. Cl.: G01N 27/327, C12Q 1/00

(54) **NOVEL BIOSENSING TECHNOLOGY BASED ON ENZYMATIC ELECTROCHEMICAL IMPEDANCE MEASUREMENT**
NEUE BIOSENSING TECHNIK BASIERT AUF ENZYMATISCH ELEKTROCHEMISCHEM IMPEDANCE MESSUNGEN
NOUVELLE BIOSENSING TECHNOLOGIE BASÉ SUR UNE MESURAGE D'ENZYMATIQUE ÉLECTROCHIMIQUE IMPEDANCE

(30) Priority: 25.08.2017 JP 2017162252
(43) Date of publication of application: 27.02.2019
(73) Proprietor: ARKRAY, Inc., Kyoto-shi, Kyoto 601-8045 (JP); Ultizyme International Ltd., Meguro-ku, Tokyo 152-0013 (JP)
(72) Inventor: SODE, Koji, Meguro-ku, Tokyo 152-0013 (JP); SHIMAZAKI, Junko, Meguro-ku, Tokyo 152-0013 (JP)
(74) Representative: Dehns

(56) References cited:
- EP-A1- 3 156 788
- WO-A1-2016/182315
- WO-A2-2004/079848
- US-A1- 2013 060 105
- US-A1- 2015 164 371
- US-A1- 2015 176 049
- VIDAKOVIC-KOCH T ET AL: "Application of electrochemical impedance spectroscopy for studying of enzyme kinetics", ELECTROCHIMICA ACTA, ELSEVIER, AMSTERDAM, NL, vol. 110, 13 March 2013 (2013-03-13), pages 94-104, XP028767592, ISSN: 0013-4686, DOI: 10.1016/J.ELECTACTA.2013.03.026
- YUKA ITO ET AL: "Third generation impedimetric sensor employing direct electron transfer type glucose dehydrogenase", BIOSENSORS AND BIOELECTRONICS, vol. 129, 1 March 2019 (2019-03-01), - 1 March 2019 (2019-03-01), pages 189-197, XP055692796, AMSTERDAM, NL ISSN: 0956-5663, DOI: 10.1016/j.bios.2019.01.018

## Description

### TECHNICAL FIELD

The present invention relates to an enzymatic electrochemical sensing technology for quantification of a substance such as glucose.

### BACKGROUND ART

An enzyme electrode used for a biosensor has a structure that extracts electrons generated by an enzyme reaction from an electrode, and in general, it includes the electrode, and a reagent layer formed by immobilization of an enzyme and conductive particles on the surface of the electrode using a cross-linking agent or a binder. The conventional methods have been mainly based on measurement of the concentration of a substrate for an enzyme reaction using as an index the enzymatic catalytic reaction current caused by oxidation-reduction reaction using an electron transfer mediator. Recently, however, a biosensor using a "direct electron transfer-type enzyme electrode" has been developed. In this biosensor, electrons generated by an enzyme reaction are directly transferred to the electrode without involvement of an oxidation-reduction substance such as an electron transfer mediator, thereby achieving electron transfer between the enzyme and the electrode. For example, Patent Document 1 discloses an enzyme sensor comprising an enzyme electrode having a detection layer containing an oxidoreductase, a water-soluble conductive polymer, and a conductive particle, wherein electron transfer occurs between the enzyme and the electrode by direct electron transfer in the detection layer.

[Patent Document 1] WO 2014/002999

[Non-patent Document 1] "Direct Electron Transfer Type Disposable Sensor Strip for Glucose Sensing Employing an Engineered FAD Glucose Dehydrogenase", Yuki Yamashita, Stefano Ferri, Mai Linh Huynh, Hitomi Shimizu, Hideaki Yamaoka, and Koji SODE, Enzyme and Microbial Technology, Volume 52, Issue 2, 5 February 2013, Pages 123-128
[Non-patent Document 2] "The Application of Engineered Glucose Dehydrogenase to a Direct Electron- Transfer - Type Continuous Glucose Monitoring System and a Compartmentless Biofuel Cell" Junko Okuda, Mieko Fukasawa, Noriko Kakehi, Tomohiko Yamazaki and Koji SODE, Anal.Lett., 40(3), 431-440 (2007)
[Non-patent Document 3] "Construction and Characterization of Direct Electron Transfer-Type Continuous Glucose Monitoring System Employing Thermostable Glucose Dehydrogenase Complex", Tomohiko Yamazaki, Junko Okuda-Shimazaki, Chikako Sakata, Taiki Tsuya, and Koji SODE, Analytical Letters, 41(13), 2363-2373(2008)

### SUMMARY OF THE INVENTION

Non-patent Documents 1, 2, and 3 propose principles of direct electron transfer-type biosensors wherein an electric potential is applied in a DC circuit using an electrode on which an oxidoreductase having an electron transfer subunit or an electron transfer domain is immobilized, thereby measuring the electric current value. In the method described in Patent Document 1, an electrode having an enzyme placed in the vicinity thereof through a conductive polymer is used. However, the method is based on amperometric measurement, and therefore has a problem that the measurement accuracy is insufficient due to the effect of diffusion of the substrate.

In view of this, at least preferred embodiments of the present invention provide an electrochemical quantification method for a substance such as glucose, which method is less likely to be affected by substrate diffusion and with which stable measurement results can be obtained.

The present inventors found that, in a direct electron transfer-type biosensor having an electrode to which an enzyme having an electron transfer subunit or an oxidoreductase modified with an artificial electron acceptor is immobilized, when sine waves having varying frequencies are applied together with an overvoltage (direct current (DC) bias) higher than the oxidation-reduction potential of the electron transfer subunit or the artificial electron acceptor, the resulting impedance value such as charge transfer resistance correlates with the concentration of the substance in the sample. That is, the present inventors found that impedance measurement with a direct electron transfer-type enzyme sensor enables quantification of a target substance.

One aspect of the present invention provides a method for quantifying a substance, comprising:
introducing a sample containing a measurement target substance to a biosensor comprising an enzyme electrode comprising an electrode and an oxidoreductase placed on the electrode in a state where direct electron transfer with the electrode occurs without involvement of an electron transfer mediator; and a counter electrode;
applying an AC voltage to the enzyme electrode to carry out impedance measurement; and
calculating the concentration of the substance based on an index obtained by the impedance measurement.

Preferably, the impedance measurement is carried out by applying AC voltage together with a certain DC bias voltage to the enzyme electrode.

The AC voltage may be applied as sine waves having one or more frequencies.

The AC voltage is preferably applied by changing frequencies between the first frequency of 0.1mHz to 100mHz and the second frequency of 10kHz to 1MHz.

Preferably, the index obtained by the impedance measurement is a charge transfer resistance value (Rct).

The concentration of the substance may be measured based on a calculation formula or a calibration curve which shows the correlation between 1/Rct and the concentration of the substance.

Preferably, the oxidoreductase is an oxidoreductase containing an electron transfer subunit or an electron transfer domain.

The electron transfer subunit or the electron transfer domain may contain a cytochrome.

The oxidoreductase may be an oxidoreductase modified with an artificial electron acceptor, or the electrode may be modified with an artificial electron acceptor.

In the enzyme electrode, the oxidoreductase may be immobilized on the electrode through a monolayer-forming molecule.

The monolayer-forming molecule is preferably a molecule containing a thiol group or a dithiol group, or a molecule containing pyrene.

The substance may be glucose, and the oxidoreductase may be glucose oxidoreductase.

An exemplary apparatus for quantifying a substance comprises:
a biosensor comprising:
   an enzyme electrode comprising an electrode and an oxidoreductase placed on the electrode in a state where direct electron transfer with the electrode occurs without involvement of an electron transfer mediator; and
   a counter electrode;
a control section for controlling the AC voltage to be applied to the enzyme electrode of the biosensor;
a measurement section for measuring impedance obtained by the application of the AC voltage;
an arithmetic section for calculating the concentration of the measurement target substance based on an index obtained by the impedance measurement; and
an output section for outputting the concentration of the measurement target substance calculated.

Preferably, the control section also controls an application of a certain DC bias voltage to the enzyme electrode.

Preferably, the index obtained by the impedance measurement is a charge transfer resistance value (Rct), and the measurement section calculates the concentration of the measurement target substance based on a calculation formula or a calibration curve which shows the correlation between 1/Rct and the concentration of the substance.

According to at least preferred embodiments of the present invention, a substance can be quantified using an electrochemical biosensor based on impedance measurement, which contributes to the fields of analysis, diagnosis, medicine, and the like. Since the charge transfer resistance value can be determined independently of the low-frequency region where mass transfer rate limitation is observed, the method based on impedance measurement enables accurate quantification of a measurement target substance without being affected by factors that inhibit mass transfer, such as the diffusion coefficient of the measurement target substance, the viscosity of the measurement solution, or the presence of particles.

### BRIEF DESCRIPTION OF THE DRAWINGS

Fig. 1 is a schematic diagram illustrating the structure of an enzyme electrode that can be used in one embodiment of the present invention;
Fig. 2 is a process chart illustrating one example of a method for producing a biosensor according to one embodiment of the present invention, and (a) to (e) illustrate schematic diagrams of the biosensor in respective steps;
Fig. 3 is a schematic diagram illustrating an exemplary measuring apparatus;
Fig. 4 is a flow chart illustrating one embodiment of a measurement program using an exemplary measuring apparatus;
Fig. 5 is a Nyquist diagram based on the result of the frequency response analysis in Example 1;
Fig. 6 is an example of an equivalent circuit for analysis of the impedance between electrodes;
Fig. 7 is the relationship between the glucose concentration and the charge transfer resistance in Example 1; and
Fig. 8 is a Nyquist diagram based on the result of the frequency response analysis in Example 2.

### EMBODIMENTS FOR CARRYING OUT THE INVENTION

A method for quantifying a substance as an embodiment of the present invention is described below with reference to drawings and the like. Each embodiment described below is merely an example, and the present invention is not limited to the following embodiments.

The method for quantifying a substance according to one embodiment of the present invention comprises the steps of:
introducing a sample containing a measurement target substance to a biosensor comprising an enzyme electrode comprising an electrode and an oxidoreductase placed on the electrode in a state where direct electron transfer with the electrode occur; and a counter electrode;
applying an AC voltage to the enzyme electrode to carry out impedance measurement; and
calculating the concentration of the substance based on an index obtained by the impedance measurement.

By the method according to one embodiment of the present invention, the concentration of a measurement target substance (for example, glucose) contained in a sample can be measured based on impedance measurement. The sample is not limited as long as it contains the measurement target substance. The sample may be a biological sample, and examples thereof include blood and urine.

### (Biosensor)

An exemplary biosensor to be used in the method according to one embodiment of the present invention comprises: an enzyme electrode (working electrode) comprising an electrode and an oxidoreductase placed on the electrode in a state where direct electron transfer with the electrode occurs; and a counter electrode. The biosensor may also be a three-electrode system comprising an enzyme electrode, a counter electrode, and a reference electrode. The counter electrode may be one that can be generally used as a counter electrode of a biosensor. Examples of the counter electrode include a carbon electrode prepared in the form of a film by screen printing; a metal electrode prepared in the form of a film by physical vapor deposition (PVD, for example, sputtering) or chemical vapor deposition (CVD); and a silver/silver chloride electrode prepared in the form of a film by screen printing. Similarly, the reference electrode may be a silver/silver chloride electrode, carbon electrode, metal electrode, or the like.

### (Enzyme Electrode)

The enzyme electrode comprises an oxidoreductase placed on the electrode in a state where direct electron transfer with the electrode occurs.

The electrode is formed using a metallic material or a carbon material, wherein examples of the metallic material include gold (Au), platinum (Pt), silver (Ag), and palladium (Pd), and representative examples of the carbon material include carbons such as graphites, carbon nanotubes, graphene, and mesoporous carbon. The electrode may be provided on an insulating substrate formed with an insulating material, wherein examples of the insulating material include resins (plastics) such as thermoplastic resins including polyetherimide (PEI), polyethylene terephthalate (PET), and polyethylene (PE), as well as polyimide resins and epoxy resins; glasses; ceramics; and papers.

### (Oxidoreductase)

Any oxidoreductase can be used as long as the oxidoreductase is capable of direct electron transfer with the electrode, or as long as the oxidoreductase becomes capable of electron transfer when the enzyme is modified with an artificial electron acceptor or a nanomaterial, or when an artificial electron acceptor or a nanomaterial is used as a material of the electrode.

Examples of the oxidoreductase include glucose oxidase (GOD), galactose oxidase, bilirubin oxidase, pyruvate oxidase, D- or L-amino acid oxidase, amine oxidase, cholesterol oxidase, choline oxidase, xanthine oxidase, sarcosine oxidase, L-lactate oxidase, ascorbate oxidase, alcohol dehydrogenase, glutamate dehydrogenase, cholesterol dehydrogenase, aldehyde dehydrogenase, glucose dehydrogenase (GDH), fructose dehydrogenase, sorbitol dehydrogenase, lactate dehydrogenase, malate dehydrogenase, glycerol dehydrogenase, 17B hydroxysteroid dehydrogenase, estradiol 17B dehydrogenase, amino acid dehydrogenase, glyceraldehyde 3-phosphate dehydrogenase, 3-hydroxysteroid dehydrogenase, diaphorase, cytochrome oxidoreductase, catalase, peroxidase, and glutathione reductase. In particular, the oxidoreductase may be an oxidoreductase of a sugar such as glucose oxidoreductase, and examples of the oxidoreductase of a sugar include glucose oxidase (GOD), galactose oxidase, glucose dehydrogenase (GDH), fructose dehydrogenase, and sorbitol dehydrogenase. Thus, depending on the type of the enzyme, the biosensor may be used as a glucose sensor, cholesterol sensor, ethanol sensor, sorbitol sensor, fructose sensor, cellobiose sensor, lactate sensor, uric acid sensor, or the like.

Examples of the oxidoreductase capable of direct electron transfer with the electrode include oxidoreductases inherently containing an oxidation-reduction molecule involved in electron transfer with the electrode. For example, an oxidoreductase containing an electron transfer subunit or an electron transfer domain as the oxidation-reduction molecule may be used. Examples of the electron transfer subunit include heme-containing subunits. Examples the oxidoreductase containing a heme-containing subunit include those containing a cytochrome such as cytochrome c or cytochrome b.

Examples of the enzyme containing a cytochrome-containing subunit as an electron transfer subunit include glucose dehydrogenase (GDH), sorbitol dehydrogenase (Sorbitol DH), D-fructose dehydrogenase (Fructose DH), lactate dehydrogenase, and urate oxidase.

Examples of the glucose dehydrogenase containing a cytochrome include cytochrome glucose dehydrogenase (CyGDH), which has a catalytic α-subunit containing FAD. Examples of the CyGDH include FAD-dependent glucose dehydrogenase derived from *Burkholderia cepacia,* and mutants thereof. Examples of the mutants of FAD-dependent glucose dehydrogenase derived from *Burkholderia cepacia* include one which has a mutant α-subunit in which the amino acid residues at positions 472 and 475 are substituted (for example, as disclosed in WO 2005/103248), a mutant α-subunit in which the amino acid residues at positions 326, 365, and 472 are substituted (for example, as disclosed in JP 2012-090563 A), and a mutant α-subunit in which the amino acid residues at positions 365, 326, 472, 475, 529, and the like are substituted (for example, as disclosed in WO 2006/137283).

Examples of the enzyme containing an electron transfer domain include enzymes containing a heme domain or a cytochrome domain. Specific examples such an enzyme include quinoheme ethanol dehydrogenase (QHEDH, PQQ Ethanol dh). Examples of the enzyme containing a cytochrome-containing domain as an electron transfer domain include "QHGDH" (fusion enzyme; GDH with heme domain of QHGDH) and cellobiose dehydrogenase. The fusion protein of PQQ glucose dehydrogenase (PQQGDH) and cytochrome disclosed in WO 2005/030807 may also be used.

On the other hand, it has been reported that, in cases where the oxidoreductase is an enzyme incapable of electron transfer with the electrode by itself, that is, in cases where the oxidoreductase does not contain an electron transfer subunit or an electron transfer domain, direct electron transfer between the oxidoreductase and the electrode becomes possible when the oxidoreductase is modified with an artificial electron acceptor or a nanomaterial, or when the electrode is modified with an artificial electron acceptor or a nanomaterial, so that an enzyme electrode capable of direct electron transfer can be constructed. Such an embodiment is also applicable to the method of the present invention.

The artificial electron acceptor herein may be a compound having no catalytic action which receives an electron from an oxidoreductase to undergo reduction, and is then reoxidized by the electrode. Examples of the artificial electron acceptor include quinone compounds (for example, 1,4-naphthoquinone, VK3, 9,10-phenanthrenequinone, 1,2-naphthoquinone, p-xyloquinone, methylbenzoquinone, 2,6-dimethylbenzoquinone, sodium 1,2-naphthoquinone-4-sulfonate, 1,4-anthraquinone, tetramethylbenzoquinone, and thymoquinone), phenylenediamine compounds (for example, N,N-dimethyl-1,4-phenylenediamine and N,N,N',N'-tetramethyl-1,4-phenylenediamine dihydrochloride), 1-methoxy-PMS (1-methoxy-5-methylphenazinium methylsulfate), PES (phenazine ethosulfate), coenzyme Q0, AZURE A chloride, phenosafranin, 6-aminoquinoxaline, and tetrathiafulvalene.

Examples of the method for the modification of the oxidoreductase with the artificial electron acceptor include a method in which the artificial electron acceptor is chemically bound to the enzyme. For example, the method may be a method in which a functional group such as succinimide is introduced to the artificial electron acceptor, and the functional group is then reacted with an amino group of the enzyme to introduce the artificial electron acceptor to the enzyme.

As a nanomaterial, an electrically conductive material that can be used to place the enzyme at a distance at which direct electron transfer from the active center of the enzyme to the electrode occurs may be used. Examples of the nanomaterial include carbon nanotubes (for example, as disclosed in Analytical Biochemistry, Volume 332, Issue 1, 1 September 2004, Pages 75-83) and metal nanoparticles (for example, as disclosed in Analytical Biochemistry Volume 331, Issue 1, 1 August 2004, Pages 89-97). However, the nanomaterial is not limited thereto as long as direct electron transfer can be observed.

The "direct electron transfer-type enzyme electrode" is a type of enzyme electrode in which electrons generated by enzyme reaction in a reagent layer are directly transferred to an electrode without involvement of, for example, the diffusion action of an oxidation-reduction substance such as an electron transfer mediator, thereby achieving electron transfer between the enzyme and the electrode.

To provide a direct electron transfer-type enzyme electrode, it is important to place the oxidoreductase in the vicinity of the electrode. Since the upper limit of the distance at which direct electron transfer occurs in a physiological reaction system is said to be 10 to 20 Å, it is important to place the enzyme at a distance shorter than this from the electrode so that electron transfer from the enzyme to the electrode is not prevented. The method for placing the enzyme at such a distance is not limited, and examples of the method include a method in which the oxidoreductase is chemically immobilized on the electrode, a method in which the oxidoreductase is indirectly immobilized on the electrode using a conductive polymer, cross-linking agent, or the like (for example, as disclosed in the Patent Document 1 described above, or JP 2016-121989 A), and a method in which the enzyme is immobilized on the electrode through a monolayer-forming molecule.

The method can be the following method in which the enzyme is immobilized on the electrode through a monolayer-forming molecule.

### (Monolayer-forming Molecule)

The monolayer-forming molecule may be a compound capable of binding to an electrode surface and bonding an enzyme molecule, which compound is also capable of forming a monolayer by bonding plurally to the electrode surface in a certain direction. The monolayer-forming molecule may have a first functional group having affinity for the electrode, a spacer region, and a second functional group capable of reacting with a functional group of the enzyme molecule. The monolayer-forming molecule may have a structure in which the first functional group having affinity for the electrode is bound to a first end of the spacer region, and the second functional group capable of reacting with the functional group of the enzyme molecule is bound to a second end of the spacer region.

The first functional group having affinity for the electrode is appropriately selected depending on the type of electrode.

Examples of the binding mode of the monolayer-forming molecule to the electrode surface include chemical bonds such as a covalent bond, coordinate bond, and ionic bond; and physical bonds by van der Waals force or the like. The binding mode may be a covalent bond or a coordinate bond. Examples of the first functional group capable of binding to the electrode include a thiol group and a dithiol group in cases of a metal electrode. On the other hand, in cases of a carbon electrode, examples of the first functional group include pyrene and porphyrin.

The second functional group capable of reacting with the functional group of the enzyme molecule is appropriately selected depending on the type of the functional group of the enzyme molecule. In cases of reaction with an amino group of the enzyme molecule (including a terminal amino group and side chain amino groups), the second functional group may be, for example, a succinimide group. In such cases, the second end of the monolayer-forming molecule turns into a reaction residue of the succinimide group and the amino group. In cases of reaction with a carboxyl group of the enzyme molecule (including a terminal carboxyl group and a side chain carboxyl group), the second functional group may be, for example, an oxazoline group. In such cases, the second end of the monolayer-forming molecule turns into a reaction residue of the oxazoline group and the carboxyl group. Thus, by the reaction between the enzyme and the second functional group, the enzyme can be immobilized to the monolayer-forming molecule by covalent bonding.

In order for the enzyme electrode to be a "direct electron transfer-type enzyme electrode", the length of the spacer may be a length with which the enzyme molecule can be kept within a certain distance from the electrode (current collector) surface. As described above, the upper limit of the distance at which direct electron transfer occurs in a physiological reaction system is said to be 10 to 20 Å. Also in the case of electron transfer in an electrochemical reaction system including an electrode and an enzyme, detection of electron transfer on the electrode is difficult at a distance longer than this without involvement of transfer (for example, transfer by diffusion) of a mediator. Thus, the length may be a length with which the enzyme can be kept within a distance of 20 Å from the electrode, or a length with which the enzyme can be kept within a distance of 10 Å from the electrode. Examples of the type of the spacer include C₁-C₂₀ (C₁-C₁₀, or C₁-C₅) alkylene, C₁-C₂₀ (C₁-C₁₀, or C₁-C₅) alkenylene, C₁-C₂₀ (C₁-C₁₀, or C₁-C₅) alkynylene, polyethylene glycols with degrees of polymerization of 2 to 50 (2 to 10, or 2 to 5), and oligopeptides having 1 to 20 (1 to 10, or 1 to 5) amino acid residues. The alkylene, alkenylene, and alkynylene may have partial substitution of -CH₂- with -S-, or partial substitution of non-consecutive -CH₂- with -O-.

Examples of the monolayer-forming molecule having a thiol group or dithiol group include compounds having the following structure. These are monolayer-forming compounds.

L is a spacer, and X is a functional group capable of reacting with a functional group of the enzyme molecule.

SH-L-X ... (1)

X-L-S-S-L-X ... (2)

Examples of such compounds include DSH, which is shown below.

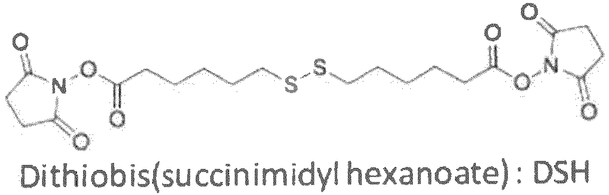

In cases of such a monolayer-forming molecule having dithiol, two enzyme molecules can be bound per one monolayer-forming molecule.

Examples of the monolayer-forming molecule having pyrene or porphyrin include compounds having the following structure.

Py is pyrene; Po is porphyrin; L is a spacer; and X is a functional group capable of reacting with a functional group of the enzyme molecule.

Py-L-X ... (3)

Po-L-X ... (3')

Examples of the monolayer-forming molecule having pyrene include compounds having the following structure. 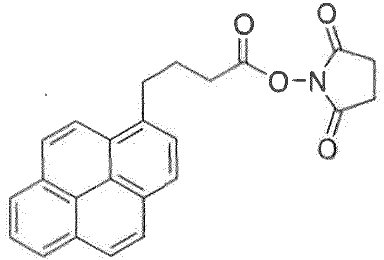 1-pyrenebutanoic acid succinimidyl ester: PySE (As disclosed in J. Am. Chem. Soc. 2001, 123, 3838-3839, Noncovalent Sidewall Functionalization of Single-Walled Carbon Nanotubes for Protein, and
J. Electroanal. Chem., 1994, 365, 157-164, Application of bifunctional reagents for immobilization of proteins on a carbon electrode surface: Oriented immobilization of photosynthetic reaction centers.)

Examples of the molecule which has pyrene and forms a monolayer include the following compound.

PHT, exemplified below, has SH at its end. A succinimide group, for example, may be added to the SH group using N-(6-maleimidocaproyloxy)succinimide or the like to allow reaction with an amino group of the enzyme, or an enzyme to which a maleimide group is introduced may be used to immobilize the enzyme to PHT through the maleimide group. 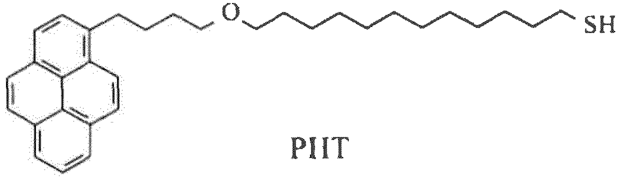 17-(1-pyrenyl)-13-oxo-heptadecanethiol: PHT (As disclosed in Chemical Physics Letters 367 (2003) 747-752,
Self-assembly of gold nanoparticles to carbon nanotubes using a thiol-terminated pyrene as interlinker.)

### (Method for Preparing Enzyme Electrode)

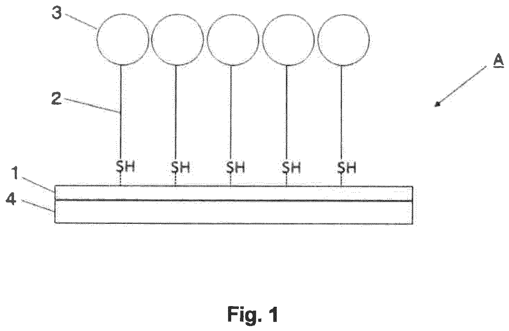

An enzyme electrode A is prepared, for example, as follows. More specifically, as shown in Fig. 1, a metal layer which functions as an electrode 1 is formed on one side of an insulating substrate 4. For example, a metal layer having a desired thickness (for example, about 30 nm) is formed by depositing a metallic material, by physical vapor deposition (PVD, for example, sputtering) or chemical vapor deposition (CVD), on one side of the insulating substrate 4 in the form of a film having a predetermined thickness (for example, about 100 µm). Instead of the metal layer, an electrode layer made of a carbon material may be formed.

To the surface of the thus obtained electrode layer, an enzyme is bound.

For example, in cases where a monolayer-forming molecule is used, first, a monolayer-forming molecule 2 is bound onto the electrode 1. Thereafter, by reacting a reactive functional group of the monolayer-forming molecule with an amino group or a carboxyl group of an oxidoreductase 3, the oxidoreductase 3 can be immobilized on the electrode 1 through the monolayer-forming molecule.

In cases where the enzyme is immobilized onto the electrode using a conductive polymer or a cross-linking agent, the enzyme and a reagent(s) such as the conductive polymer or the cross-linking reagent may be added onto the electrode 1 to prepare an enzyme electrode.

### (Biosensor)

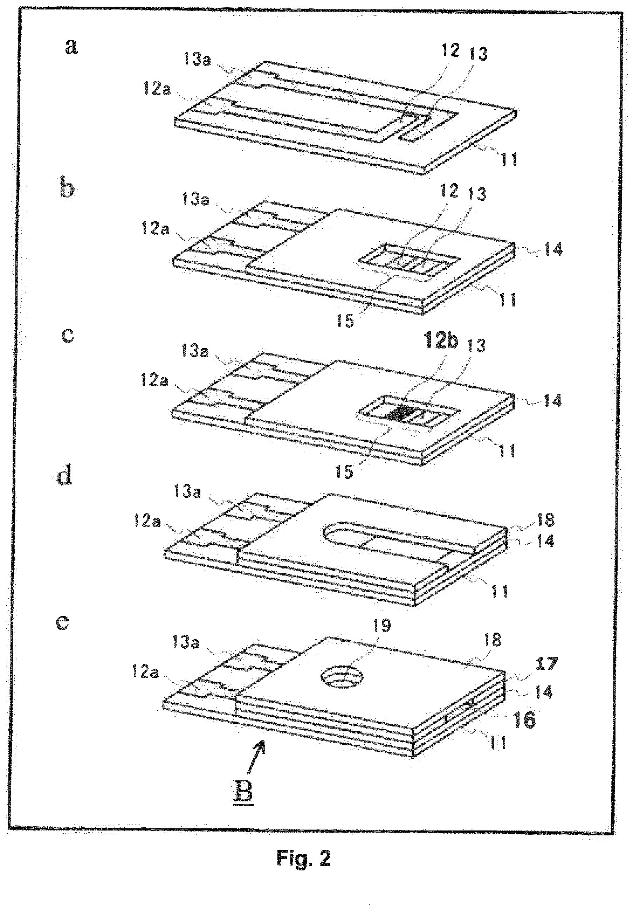

Hereinafter, one example of the biosensor will be described based on Fig. 2, which illustrates a series of steps of producing the biosensor. The biosensor to be used in the present invention is not limited to the following embodiment.

As illustrated in Fig. 2, a biosensor B includes a base plate 11, an electrode pair composed of a working electrode 12 having a lead section 12a and a counter electrode 13 having a lead section 13a, an insulating layer 14, a reagent layer 12b comprising an oxidoreductase, a spacer 17 having an opening, and a cover 18 having a through-hole 19. As illustrated in Fig. 2b, a detection section 15 is provided on the base plate 11, and the working electrode 12 and the counter electrode 13 are disposed on the detection section 15 in parallel with the width direction of the base plate 11. Each one end of the electrode pair serves as each of the lead sections 12a and 13a and is disposed so as to be perpendicular to each other end on the detection section 15. A region between the working electrode 12 and the counter electrode 13 corresponds to an insulating section. As illustrated in Fig. 2b, the insulating layer 14, excluding the lead sections 12a and 13a and the detection section 15, is laminated on the base plate 11 provided with such an electrode system, and the reagent layer 12b is provided on the detection section 15 on which the insulating layer 14 is not laminated. As illustrated in Fig. 2d, a spacer 17 having an opening in a region corresponding to the detection section 15 is then disposed on the insulating layer 14. A cover 18 having a through-hole 19, which is provided on a location corresponding to a portion of the opening of the spacer, is disposed on the spacer 17 (Fig. 2e). In such a biosensor B, a space portion of the opening, which is a space portion sandwiched among the reagent layer 12b and the insulating layer 14, and the cover 18, serves as a sample-feeding section 16 of a capillary structure. The through-hole 19 then serves as an air hole for sucking the sample by a capillary phenomenon.

### (Method for Quantifying Substance)

The quantification method according to one embodiment of the present invention comprises:
applying an AC voltage to the enzyme electrode to carry out impedance measurement; and
calculating the concentration of the substance based on an index obtained by the impedance measurement.

The impedance measurement may be carried out by a method in which sine waves having one or more frequencies are applied together with a certain DC bias voltage to the enzyme electrode.

The DC bias may be a voltage higher than the oxidation-reduction potential of the oxidation-reduction molecule (electron transfer subunit, electron transfer domain, or artificial electron acceptor) involved in the direct electron transfer with the electrode. The upper limit of the voltage to be applied as DC bias is not limited, and may be about +1000mV.

For example, in the case of using an oxidoreductase having heme (cytochrome) as an electron transfer subunit or an electron transfer domain, the voltage to be applied as DC bias may be +300 mV or higher. In cases where PES is used, the voltage to be applied as DC bias may be, for example, +100mV or higher.

More specifically, for example, first, the DC bias voltage is applied between the working electrode and the counter electrode. Subsequently, a voltage generated by superposition of an AC voltage on the DC bias voltage is applied to the working electrode. The AC voltage to be applied is preferably as low as possible at which the impedance between the working electrode and the counter electrode can be measured. In cases where the value of the AC voltage applied to the working electrode is too high, electrochemical reaction occurs on the surface of the electrode, causing problems such as changes in the surface conditions of the electrode. In cases where the value of the AC voltage is too low, the SN ratio decreases, leading to inaccurate measurement of the impedance. Thus, more specifically, the voltage applied between the working electrode and the counter electrode may have amplitude of 5 to 20 mV.

Subsequently, using a frequency response analyzer, the impedance between the working electrode and the counter electrode is measured while changing the frequency of the AC voltage applied. For example, the frequency can be changed between the first frequency (lower limit) of 0.1mHz to 100mHz and the second frequency (upper limit) of 10kHz to 1MHz, and more specifically, an appropriate range of the varying frequency is from 0.1 mHz to 100 kHz. However, the lower the frequency range, the longer length of time the measurement of the impedance requires, leading to long measurement intervals. Thus, the lower limit of the frequency may be about 1 mHz, or about 10 mHz, or about 100mHz.

The upper limit of the frequency may be about 10kHz, or about 100kHz, or about 1MHz.

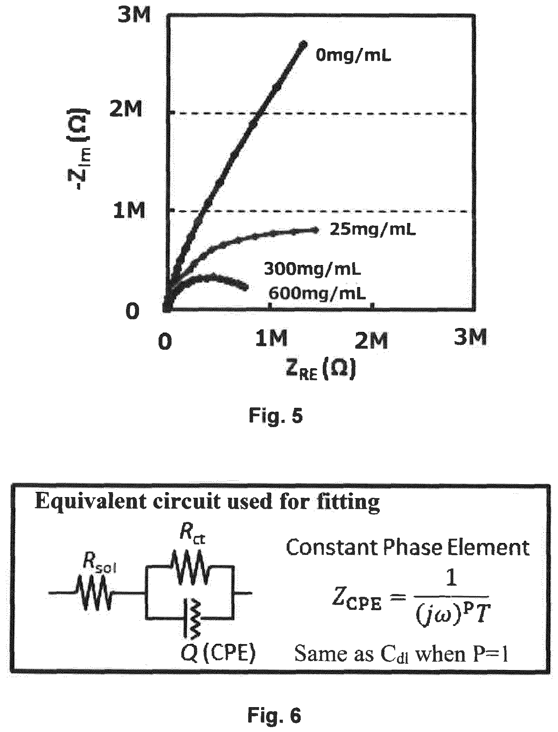

An example of the index obtained by the impedance measurement is the charge transfer resistance value (Rct). The charge transfer resistance value (Rct) can be calculated by, for example, drawing a Nyquist diagram such as the one shown in Fig. 5 based on the data obtained with the frequency response analyzer, and then fitting the diagram using an equivalent circuit such as the one shown in Fig. 6.

Fig. 6 is an equivalent circuit for analysis of the impedance between electrodes. In Fig. 6, Rsol represents the conductive resistance between a working electrode and a counter electrode; Q (CPE) represents the electric capacity on the electrode surface; and Rct represents the resistance of the enzyme electrode reaction. The other terms in Fig. 6 are as follows: omega: angular frequency
j: imaginary unit
P: CPE exponent
T: CPE constant
Cdl: Double layer capacity

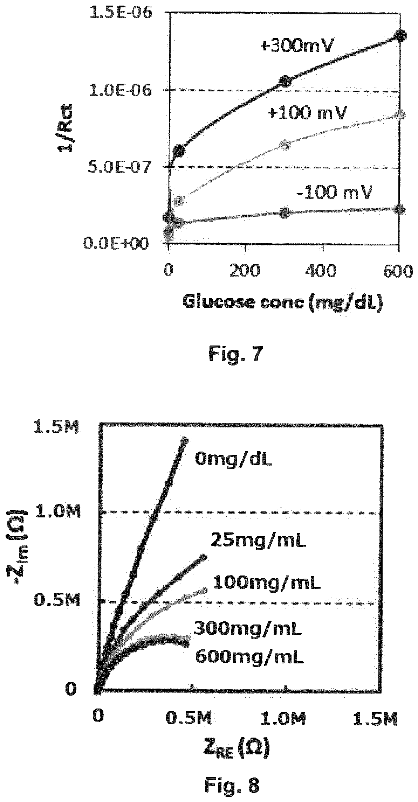

Subsequently, the concentration of the substance is calculated based on a value such as the charge transfer resistance value (Rct). The charge transfer resistance value is dependent on the concentration of the substance in the sample. As the concentration of the substance increases, the resistance decreases. By preliminarily preparing a property diagram showing the correlation between the reciprocal of the resistance Rct and the concentration of the substance as shown in Fig. 7, and using this property diagram, the concentration of the substance can be calculated based on the calculated Rct.

### (Apparatus)

An exemplary measuring apparatus is described below with reference to drawings.

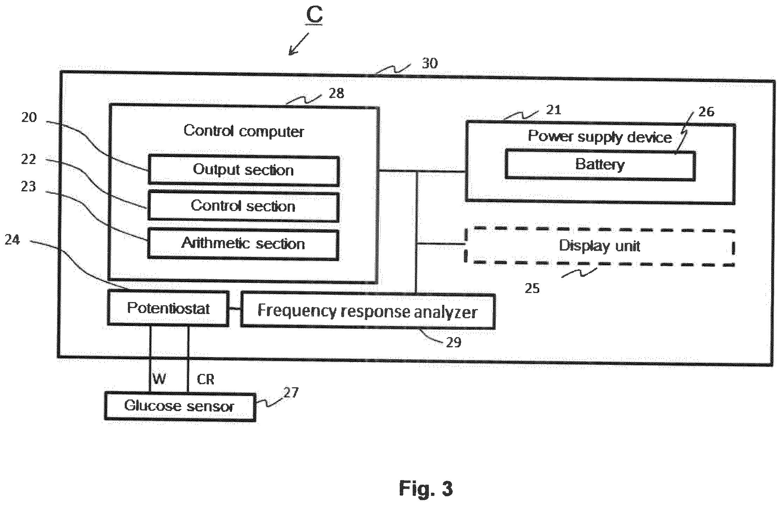

Fig. 3 shows an example of the configuration of main electronic components included in a measuring apparatus C. A control computer 28, a potentiostat 24, a frequency response analyzer 29, and a power supply device 21 are provided on a base 30 housed in a housing.

The control computer 28 includes, as hardware, a processor such as CPU (central processing unit); a recording medium such as a memory (RAM (Random Access Memory) or ROM (Read Only Memory)); and a communication unit. When the processor loads a program stored in the recording medium (for example, the ROM) to the RAM, and executes the program, the control computer 28 functions as an apparatus including an output section 20, a control section 22, an arithmetic section 23, and a measurement section (the potentiostat 24 and the frequency response analyzer 29). The control computer 28 may also include an auxiliary memory such as a semiconductor memory (EEPROM or flash memory) or a hard disk.

The control section 22 controls, for example, the timing for applying the voltage and the value of the voltage to be applied.

The power supply device 21 includes a battery 26, and supplies electricity to the control computer 28, the potentiostat 24, and the frequency response analyzer 29, to allow their operation. The power supply device 21 may also be arranged outside the housing.

The potentiostat 24 is a device which keeps the potential of the working electrode constant with respect to the potential of the reference electrode. The potentiostat 24, which is controlled by the control section 22, applies a predetermined amount of voltage (DC bias) between a counter electrode and a working electrode of a glucose sensor 27 using terminals CR and W, and superposes an AC voltage on the working electrode.

The frequency response analyzer 29, which is controlled by the control section 22, changes the frequency of the AC voltage applied to the working electrode, and measures the impedance between the working electrode and the counter electrode. That is, the frequency response analyzer 29 includes a sine-wave oscillator, and an analyzer (analysis device) for measuring the intensity and the phase of the response signal. The frequency response analyzer 29 is capable of applying sine waves to the battery from the oscillator, retrieving the response signal from the battery, and then converting the response signal into digital data to detect the amplitude and the phase at the measurement frequency.

The arithmetic section 23 calculates the index such as charge transfer resistance based on the detected impedance, and then calculates the concentration of the measurement target substance based on the index such as charge transfer resistance, followed by storing the calculated result. The output section 20 carries out data communication with a display unit 25, and sends the calculated result on the concentration of the measurement target substance provided by the arithmetic section 23 to the display unit 25. The display unit 25 is capable of displaying, for example, the calculated result on the glucose concentration received from the measuring apparatus C, on a display screen in a predetermined format.

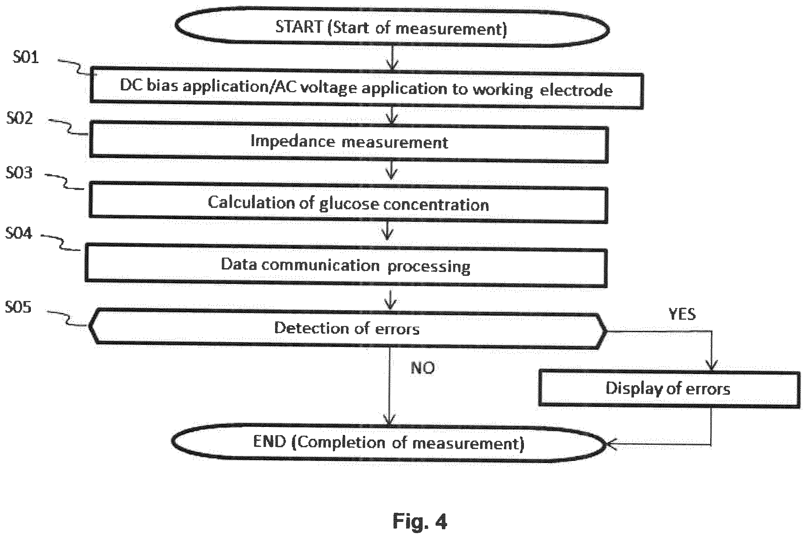

Fig. 4 is a flow chart illustrating an example of the glucose concentration measurement process carried out by the control computer 28. When the CPU (control section 22) of the control computer 28 receives an instruction to start the measurement of the glucose concentration, the control section 22 controls the potentiostat 24 to apply a predetermined amount of DC bias voltage to the working electrode. By controlling the frequency response analyzer 29 to superpose an AC voltage thereon, the measurement is started (Step S01).

The frequency response analyzer 29 then measures the impedance between the working electrode and the counter electrode while changing the frequency of the AC voltage applied to the working electrode. The frequency response analyzer 29 then sends the measurement result on the impedance to the arithmetic section 24 (Step S02).

The arithmetic section 23 carries out arithmetic processing based on the impedance, and calculates the glucose concentration (Step S03).

For example, the arithmetic section 23 prepares a Nyquist diagram based on the input impedance, and analyzes the charge transfer resistance based on the equivalent circuit shown in Fig. 6. For example, the arithmetic section 23 of the control computer 28 preliminarily has calibration formula or calibration curve data on the reciprocal of the charge transfer resistance and the glucose concentration applicable to the equivalent circuit and the glucose oxidoreductase disposed on the electrode, and calculates the glucose concentration using the calculation formula or the calibration curve.

The output section 20 sends the calculated result on the glucose concentration to the display unit 25 through a communication link provided between the output section 20 and the display unit 25 (Step S04). Thereafter, the control section 22 determines if there are any measurement errors detected (Step S05); completes the measurement if there is no error; and displays the glucose concentration on the display unit. If there are any errors, a notification of error is displayed, and then the process by the flow shown in Fig. 4 is completed. Further, the calculated result may be stored in the arithmetic section 23, and the stored result may be accessed afterwards to be displayed on the display unit for confirmation. Although the detection of measurement errors by the control section 22 (Step S05) is carried out after the calculated result is sent to the display unit 25 (Step S04) in this embodiment, these steps may be carried out in the reverse order.

### EXAMPLES

Examples of the impedance measurement are described below. However, the present invention is not limited to the embodiments of the following Examples.

### Example 1

### (Preparation of Enzyme Electrode)

An enzyme electrode was prepared by immobilizing GDH containing a cytochrome c subunit on a gold surface via a monolayer-forming molecule. As the monolayer-forming molecule, DSH, which is shown below, was used.

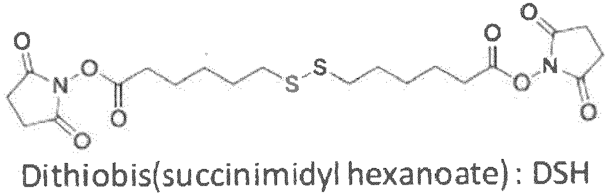

More specifically, a gold wire (diameter, 0.5 mm; length, 6 to 7 cm) was immersed in Piranha solution at room temperature overnight, and then washed with acetone. The gold wire was then immersed in a solution of DSH in acetone (concentration, 20 µM), and incubated at 25°C for 24 hours to allow binding of thiol groups of DSH to the gold surface. Subsequently, the gold wire was washed with acetone, immersed in PPB (pH 7.0) containing an enzyme (FADGDH derived from *Burkholderia cepacia* (γa(QYY)β; as disclosed in JP 2012-090563 A) (concentration 0.03 mg/ml)), and incubated at 4°C overnight to allow the enzyme to be bound via a functional group of DSH to obtain an enzyme electrode.

### (Impedance Measurement)

The impedance measurement was carried out with a three electrode system using the above enzyme electrode as a working electrode together with a counter electrode (Pt wire) and a reference electrode (silver/silver chloride). The measurement was carried out at 25°C.

To the enzyme electrode, 0 (background), 25 mg/dL, 300 mg/dL, or 600 mg/dL glucose solution was introduced as a sample, and a DC voltage of +300 mV, +100 mV, -100 mV, or -300 mV (vs. silver/silver chloride) was applied to the working electrode. In this state, sine waves (amplitude, 10 mV) were applied to the working electrode with varying frequencies (100 kHz to 50 mHz), and impedance measurement was carried out.

### (Analysis and Evaluation of Measurement Result)

The measurement result obtained when the DC voltage of +300 mV was applied to the working electrode was plotted to provide a Nyquist diagram (Fig. 5). By fitting the diagram using the equivalent circuit of Fig. 6, the charge transfer resistance (Rct) at each glucose concentration was calculated. In a similar manner, the charge transfer resistance (Rct) at each glucose concentration was calculated for the DC voltages of +100 mV and -100 mV applied to the working electrode.

Fig. 7 shows the relationship between the glucose concentration and 1/Rct determined for each applied voltage.

As a result, correlation between 1/Rct and the glucose concentration was found at +300 mV and +100 mV. However, the slope had a smaller incline at +100 mV, and no concentration dependency was found at -100 mV. Thus, it was found that 1/Rct shows a sufficient correlation with the glucose concentration when a DC voltage bias sufficient for oxidation of the heme in the electron transfer subunit is applied.

### Example 2

### (Preparation of Enzyme Electrode)

GDH to which phenazine ethosulfate (PES) is bound was immobilized on a carbon surface through a monolayer-forming molecule, to prepare an enzyme electrode. As the monolayer-forming molecule, NTA-SAM Formation Reagent (Dojindo Laboratories) was used.

More specifically, a gold wire (diameter, 0.5 mm; length, 6 to 7 cm) was immersed in Piranha solution at room temperature overnight, and then washed with acetone. The gold wire was then immersed in a solution of NTA-SAM in ethanol (concentration, 0.2 mM), and incubated at 25°C for 24 hours to allow binding of NTA-SAM to the gold surface. Subsequently, the gold wire was immersed in 40 mM aqueous NiSO₄ solution at room temperature for 1 hour to allow chelation of Ni ions by NTA. After washing, 2 µL of a Histagged mold-derived glucose dehydrogenase (BfuGDH) (concentration, 2.4 mg/ml) was added dropwise to the gold electrode modified with NTA-SAM, and the gold electrode was then dried at 25°C for 2 hours. Thereafter, 0.5 µL of PES (5 mM) having an NHS group was added to allow binding of the PES through an amino group of GDH, thereby obtaining an enzyme electrode wherein the GDH modified with the artificial electron acceptor is immobilized on the electrode through the monolayer-forming molecule (SAM).

### (Impedance Measurement)

The impedance measurement was carried out with a three electrode system using the above enzyme electrode as a working electrode together with a counter electrode (carbon) and a reference electrode (silver/silver chloride). The measurement was carried out at 25°C.

To the enzyme electrode, 0 (background), 25 mg/dL, 300 mg/dL, or 600 mg/dL glucose solution was introduced as a sample, and a DC voltage of +100 mV (vs. silver/silver chloride) was applied to the working electrode. In this state, sine waves (amplitude, 10 mV) were applied to the working electrode with varying frequencies (100 kHz to 50 mHz), and impedance measurement was carried out.

### (Analysis and Evaluation of Measurement Result)

The measurement result obtained when the DC voltage of +100mV was applied to the working electrode was plotted to provide a Nyquist diagram (Fig. 8). As a result, the charge transfer resistance was found to be dependent on the glucose concentration. When the modification with PES was not carried out, no concentration dependency was found.

### DESCRIPTION OF SYMBOLS

- A: Enzyme electrode
- 1: Electrode
- 2: Monolayer-forming molecule
- 3: Oxidoreductase
- 4: Insulating substrate

- B: biosensor
- 11: base plate
- 12: working electrode
- 12a: lead section
- 12b: reagent layer
- 13: counter electrode
- 13a: lead section
- 14: insulating layer
- 15: detection section
- 16: opening
- 17: spacer
- 18: cover
- 19: air hole

- C: Measuring apparatus
- 20: Output section
- 21: Power supply device
- 22: Control section
- 23: Arithmetic section
- 24: Potentiostat
- 25: Display unit
- 26: Battery
- 27: Glucose sensor
- 28: Control computer
- 29: Frequency response analyzer
- 30: Base
- CR, W: Terminal

## Claims

1. A method for quantifying a substance, comprising:
introducing a sample containing a measurement target substance to a biosensor (B) which comprises an enzyme electrode (12) comprising an electrode (12a) and an oxidoreductase (12b) placed on the electrode in a state where direct electron transfer with the electrode occurs without involvement of an electron transfer mediator; and a counter electrode (13);
applying an AC voltage to the enzyme electrode (12) to carry out impedance measurement; and
calculating the concentration of the substance based on an index obtained by the impedance measurement.

2. The method according to claim 1, wherein the impedance measurement is carried out by applying AC voltage together with a certain DC bias voltage to the enzyme electrode.

3. The method according to claim 1 or 2, wherein the AC voltage is applied as sine waves having one or more frequencies.

4. The method according to any preceding claim, wherein the AC voltage is applied by changing frequencies between the first frequency of 0.1mHz to 100mHz and the second frequency of 10kHz to 1MHz.

5. The method according to any preceding claim, wherein the index obtained by the impedance measurement is a charge transfer resistance value (Rct).

6. The method according to claim 5, wherein the concentration of the substance is measured based on a calculation formula or a calibration curve which shows the correlation between 1/Rct and the concentration of the substance.

7. The method according to any preceding claim, wherein the oxidoreductase is an oxidoreductase containing an electron transfer subunit or an electron transfer domain.

8. The method according to claim 7 when dependent from claim 2, wherein the DC bias voltage is higher than the oxidation/reduction potential of the electron transfer subunit or electron transfer domain.

9. The method according to claim 7 or 8, wherein the electron transfer subunit or the electron transfer domain contains a cytochrome.

10. The method according to any of claims 1 to 6, wherein the oxidoreductase is an oxidoreductase modified with an artificial electron acceptor or wherein the electrode is modified with an artificial electron acceptor.

11. The method according to claim 10 when dependent from claim 2, wherein the DC bias voltage is higher than the oxidation/reduction potential of the artificial electron acceptor.

12. The method according to any preceding claim, wherein in the enzyme electrode, the oxidoreductase is immobilized on the electrode through a monolayer-forming molecule.

13. The method according to claim 12, wherein the monolayer-forming molecule is a molecule containing a thiol group or a dithiol group, or a molecule containing pyrene.

14. The method according to any preceding claim, wherein the substance is glucose, and the oxidoreductase is glucose oxidoreductase.

## Patentansprüche

1. Verfahren zur Quantifizierung einer Substanz, umfassend:
Einführen einer Probe, die eine Messzielsubstanz enthält, in einen Biosensor (B), der eine Enzymelektrode (12), die eine Elektrode (12a) und eine Oxidoreduktase (12b) umfasst, die auf der Elektrode in einem Zustand angeordnet ist, in dem eine direkte Elektronenübertragung mit der Elektrode ohne Beteiligung eines Elektronenübertragungsmediators stattfindet, und eine Gegenelektrode (13) umfasst;
Anlegen einer Wechselspannung an die Enzymelektrode (12), um eine Impedanzmessung durchzuführen; und
Berechnen der Konzentration der Substanz auf der Grundlage eines durch die Impedanzmessung erhaltenen Index.

2. Verfahren nach Anspruch 1, wobei die Impedanzmessung durch Anlegen einer Wechselspannung zusammen mit einer bestimmten Gleichspannung an die Enzymelektrode durchgeführt wird.

3. Verfahren nach Anspruch 1 oder 2, wobei die Wechselspannung als Sinuswellen angelegt wird, die eine oder mehrere Frequenzen aufweisen.

4. Verfahren nach einem der vorstehenden Ansprüche, wobei die Wechselspannung durch Wechseln der Frequenzen zwischen der ersten Frequenz von 0,1mHz bis 100mHz und der zweiten Frequenz von 10kHz bis 1MHz angelegt wird.

5. Verfahren nach einem der vorstehenden Ansprüche, wobei der durch die Impedanzmessung erhaltene Index ein Ladungstransferwiderstandswert (Rct) ist.

6. Verfahren nach Anspruch 5, wobei die Konzentration der Substanz auf der Grundlage einer Berechnungsformel oder einer Kalibrierungskurve gemessen wird, die die Korrelation zwischen 1/Rct und der Konzentration der Substanz zeigt.

7. Verfahren nach einem der vorstehenden Ansprüche, wobei die Oxidoreduktase eine Oxidoreduktase ist, die eine Elektronentransfer-Untereinheit oder eine Elektronentransfer-Domäne enthält.

8. Verfahren nach Anspruch 7, wenn abhängig von Anspruch 2, wobei die Vorspannungsgleichspannung höher ist als das Oxidations-/Reduktionspotential der Elektronentransfer-Untereinheit oder Elektronentransfer-Domäne.

9. Verfahren nach Anspruch 7 oder 8, wobei die Elektronentransfer-Untereinheit oder die Elektronentransfer-Domäne ein Cytochrom enthält.

10. Verfahren nach einem der Ansprüche 1 bis 6, wobei die Oxidoreduktase eine mit einem künstlichen Elektronenakzeptor modifizierte Oxidoreduktase ist oder wobei die Elektrode mit einem künstlichen Elektronenakzeptor modifiziert ist.

11. Verfahren nach Anspruch 10 in Abhängigkeit von Anspruch 2, wobei die Vorspannungsgleichspannung höher ist als das Oxidations-/Reduktionspotential des künstlichen Elektronenakzeptors.

12. Verfahren nach einem der vorstehenden Ansprüche, wobei in der Enzymelektrode die Oxidoreduktase durch ein eine Monoschicht bildendes Molekül an der Elektrode immobilisiert ist.

13. Verfahren nach Anspruch 12, wobei das eine Monolage bildende Molekül ein Molekül ist, das eine Thiolgruppe oder eine Dithiolgruppe enthält, oder ein Molekül, das Pyren enthält.

14. Verfahren nach einem der vorstehenden Ansprüche, wobei die Substanz Glucose ist und die Oxidoreduktase Glucose-Oxidoreduktase ist.

## Revendications

1. Procédé de quantification d'une substance, comprenant les étapes consistant à :
introduire un échantillon contenant une substance cible de mesure dans un biocapteur (B) qui comprend une électrode enzymatique (12) comprenant une électrode (12a) et une oxydoréductase (12b) placée sur l'électrode dans un état où un transfert d'électrons direct avec l'électrode se produit sans intervention d'un médiateur de transfert d'électrons ; et une contre-électrode (13) ;
appliquer une tension de courant alternatif, CA, à l'électrode enzymatique (12) pour effectuer une mesure d'impédance ; et
calculer la concentration de la substance sur la base d'un indice obtenu par la mesure d'impédance.

2. Procédé selon la revendication 1, dans lequel la mesure d'impédance est effectuée en appliquant une tension CA avec une certaine tension de polarisation de courant continu, CC, à l'électrode enzymatique.

3. Procédé selon la revendication 1 ou 2, dans lequel la tension CA est appliquée sous forme d'ondes sinusoïdales présentant une ou plusieurs fréquences.

4. Procédé selon une quelconque revendication précédente, dans lequel la tension CA est appliquée en changeant des fréquences entre la première fréquence de 0,1 mHz à 100 mHz et la seconde fréquence de 10 kHz à 1 MHz.

5. Procédé selon une quelconque revendication précédente, dans lequel l'indice obtenu par la mesure d'impédance est une valeur de résistance de transfert de charge (Rct).

6. Procédé selon la revendication 5, dans lequel la concentration de la substance est mesurée sur la base d'une formule de calcul ou d'une courbe d'étalonnage qui montre la corrélation entre 1/Rct et la concentration de la substance.

7. Procédé selon une quelconque revendication précédente, dans lequel l'oxydoréductase est une oxydoréductase contenant une sous-unité de transfert d'électrons ou un domaine de transfert d'électrons.

8. Procédé selon la revendication 7 lorsqu'elle dépend de la revendication 2, dans lequel la tension de polarisation CC est supérieure au potentiel d'oxydation/réduction de la sous-unité de transfert d'électrons ou du domaine de transfert d'électrons.

9. Procédé selon la revendication 7 ou 8, dans lequel la sous-unité de transfert d'électrons ou le domaine de transfert d'électrons contient un cytochrome.

10. Procédé selon l'une quelconques des revendications 1 à 6, dans lequel l'oxydoréductase est une oxydoréductase modifiée avec un accepteur d'électrons artificiel ou dans lequel l'électrode est modifiée avec un accepteur d'électrons artificiel.

11. Procédé selon la revendication 10 lorsqu'elle dépend de la revendication 2, dans lequel la tension de polarisation CC est supérieure au potentiel d'oxydation/réduction de l'accepteur d'électrons artificiel.

12. Procédé selon une quelconque revendication précédente, dans lequel dans l'électrode enzymatique, l'oxydoréductase est immobilisée sur l'électrode à travers une molécule de formation de monocouche.

13. Procédé selon la revendication 12, dans lequel la molécule de formation de monocouche est une molécule contenant un groupe thiol ou un groupe dithiol, ou une molécule contenant du pyrène.

14. Procédé selon une quelconque revendication précédente, dans lequel la substance est le glucose, et l'oxydoréductase est une glucose oxydoréductase.
